Europäisches Patentamt

European Patent Office

Office européen des brevets

(11) Publication number: **0 099 126**
**B1**

# EUROPEAN PATENT SPECIFICATION

(45) Date of publication of patent specification: **29.08.90**

(51) Int. Cl.⁵: **A 61 K 37/54** // C12N9/64

(21) Application number: **83106911.7**

(22) Date of filing: **14.07.83**

(54) Thrombolytic composition.

(30) Priority: **16.07.82 JP 122981/82**

(43) Date of publication of application:
**25.01.84 Bulletin 84/04**

(45) Publication of the grant of the patent:
**29.08.90 Bulletin 90/35**

(84) Designated Contracting States:
**BE CH DE FR GB IT LI NL SE**

(56) References cited:

THE JOURNAL OF BIOLOGICAL CHEMISTRY,
vol. 254, no. 6, March 1979, pages 1998-2003;
B.R. BINDER et al.: "Purification and
characterization of human vascular
plasminogen activator derived from blood
vessel perfusates"

CHEMICAL ABSTRACTS, vol. 92, 1980, page 211,
no. 159461x, Columbus, Ohio, US; B. AASTED et
al.: "Purification and characterization of human
vascular plasminogen activator", & BIOCHIM.

(73) Proprietor: **MITSUI TOATSU CHEMICALS, Inc.**
**2-5 Kasumigaseki 3-chome**
**Chiyoda-Ku Tokyo 100 (JP)**

(72) Inventor: **Takahashi, Seishi**
**1071-2, Nakano-cho Totsuka-ku**
**Yokohama Kanagawa-ken (JP)**
Inventor: **Morimoto, Kazushi**
**4-1409-933, Shiomidai 1-chome Isogo-ku**
**Yokohama Kanagawa-ken (JP)**
Inventor: **Fujisawa, Yoshikazu**
**11-2, Tamanawa 5-chome**
**Kamakura Kanagawa-ken (JP)**
Inventor: **Ikeda, Fumiaki**
**2882, Iijima-cho Totsuka-ku**
**Yokohama Kanagawa-ken (JP)**

(74) Representative: **Schüler, Horst, Dr.**
**Kaiserstrasse 69**
**D-6000 Frankfurt/Main 1 (DE)**

(56) References cited:

BIOPHYS. ACTA 1980, 621(2), 241-54
CHEMICAL ABSTRACTS, vol. 82, 1975, page 235,
no. 2521n, Columubus, Ohio, US; M.B. BERNIK
et al.: "Immunologie identity of plasminogen
activator in human urine heart, blood vessels,
and tissue culture", & J. LAB. CLIN. MED. 1974,
84(4), 546-58

CHEMICAL ABSTRACTS, vol. 97, 27th
September 1982, page 255, no. 106145u,
Columbus, Ohio, US; K. SUEISHI et al.:
"Purification and characterization of human
kidney plasminogen activator dissimilar to
urokinase", & BIOCHIM. BIOPHYS. ACTA 1982
717(2), 327-36

# EP 0 099 126 B1

## Description

### 1. Field of the Invention:

This invention relates to a novel thrombolytic composition containing a specified plasminogen activator as the active ingredient.

### 2. Description of the Prior Art:

Thrombosis is often caused by the formation of a blood clot in a blood vessel in cases of surgical operations, delivery, trauma, infectious diseases and the like. Various attempts have been made to eliminate such a risk. One example is the administration of an anticoagulant such as heparin and coumarin derivatives. Another example is the use of a thrombolytic agent. A thrombolytic agent has the function of dissolving the blood clot formed in thrombosis and thereby removing it from the blood vessel.

Such a blood clot consists of fibrin formed from fibrinogen by the action of the enzyme thrombin. On the other hand, normal blood also contains plasminogen. When plasminogen is activated to plasmin, it dissolves fibrin by its enzymatic action and thereby breaks up the blood clot. Although the circulating blood contains all the components required to disintegrate and remove a blood clot upon its formation in a blood vessel, the potential autothrombolytic capacity of the living body, as a matter of fact, can hardly fulfil such a purpose. This is due to insufficient concentrations of the natural plasminogen activator on the blood. Accordingly, administration of an exogenous plasminogen activator is a useful means for the treatment of thrombosis.

At present, urokinase, which is a plasminogen activator isolated from urine or cultured kidney cells, and streptokinase which is a plasminogen activator derived from streptococci, are being used as thrombolytic agents. However, both urokinase and streptokinase differ from the natural plasminogen activator derived from blood and, in particular, neither of them has a specific affinity for fibrin. Therefore, the results of the use of urokinase or streptokinase in the treatment of thrombosis are not fully satisfactory in all respects. For these thrombolytic agents, relatively large doses are required to achieve the desired effect, but administration of a large dose may cause serious side effects such as decomposition of fibrinogen, internal hemorrhage and the like. Accordingly, it is strongly desired to develop a drug which can be prepared in a relatively large scale and has high thrombolytic activity accompanied with a minimum of side effects.

As a result of searching for a plasminogen activator which is closely related to the natural plasminogen activator derived from blood and has sufficient thrombolytic activity to be effective in the treatment of thrombosis, the present inventors succeeded in isolating a novel tissue plasminogen activator from the human kidneys. A plasminogen activator isolated from human kidneys and its method of purification have already been proposed elsewhere. Kwaan et al. have made a study of a tissue plasminogen activator derived from human kidneys [Fed. Proc., *24*, 387 (1965)]. Bonder et al. have disclosed a purified vascular plasminogen activator having a molecular weight ranging from 70,000 to 75,000 [J. of Biol. Chem. *254*, pp. 1998—2003, (1979)]. Further studies conducted by Kucinski et al., Bernik et al., Barlow et al., Åsted et al., and Lewis have revealed that the plasminogen activator derived from a culture of human kidney tissue is immunologically and physicochemically indentical to urokinase. In view of the side effects of urokinase which are increasingly found, the above finding has discouraged one from isolating a plasminogen activator from human kidneys and using it as a thrombolytic agent.

Nevertheless, the present inventors found unexpectedly that a tissue plasminogen activator isolated from human kidneys has properties quite different from those of urokinase. During the course of thorough investigation of this novel plasminogen activator from every point of view, its powerful thrombolytic acitivity was discovered. The present invention has been completed on the basis of this discovery.

### Summary of the Invention:

It is an object of the present invention to provide a novel thrombolytic composition having powerful thrombolytic activity.

According to the present invention, there is provided a thrombolytic composition comprising a plasminogen activiator as the active ingredient derived from human kidney and a pharmaceutically acceptable carrier. The plasminogen activator constituting the active ingredient of the thrombolytic composition of the present invention has the following characteristic properties:

(1) The principal protein band obtained by sodium dodecyl sulfate-polyacrylamide gel electrophoresis has an apparent molecular weight of $55,000\pm1,000$;

(2) The apparent molecular weight remains unchanged in the presence of a reducing agent;

(3) The principal band obtained by isoelectric-point electrophoresis has a pI in the range of 7 to 9;

(4) The plasminogen activator has the immunological property of not being adsorbed by anti-urokinase IgG-Sepharose affinity chromatography; and

(5) The plasminogen activator hydrolyzes H-D-valyl-L-leucyl-L-lysine-p-nitroanilide dihydrochloride and H-D-isoleucyl-L-propyl-L-arginine-p-nitroanilide dihydrochloride, but does not hydrolyse Boc-L-valyl-L-prolyl-L-arginine-4-methylcoumaryl-7-amide, carbobenzoxy-L-phenylalanyl-L-arginine-4-methylcoumaryl-7-amide, L-prolyl-L-phenylalanyl-L-arginine-4-methylcoumaryl-7-amide or glutarylglycyl-L-arginine-4-methylcoumaryl-7-amide.

Brief Description of the Drawings:

In the drawings:

Fig. 1 is an elution curve obtained by eluting the plasminogen activator constituting the active ingredient of the thrombolytic composition of the present invention according to the procedure described in Example 1;

Fig. 2 is a diagram comparatively illustrating the thrombolytic effects of the plasminogen activator of the present invention and urokinase; and

Fig. 3 is a diagram illustrating the thrombolytic effects of the plasminogen activator of the present invention on clots of blood obtained from man and various animals.

Detailed Description of the Invention:

The plasminogen activiator constituting the active ingredient of the thrombolytic composition of the present invention has the above-described characteristic properties. The plasminogen activator is further characterized in that essentially all of it can be adsorbed into a fibrin-Sepharose column and then eluted with 2M NH₄SCN and in that it can be obtained from kidneys or blood vessels by extraction or perfusion with 1—2M NH₄SCN (pH 7.4) and then purified in the presence of a nonionic surface active agent such as 0.1% Triton X—100. When stored at 4°C in a buffer solution of pH 7.4, it remains unchanged after 2 weeks.

The plasminogen activator constituting the active ingredient of the thrombolytic composition of the present invention can be prepared, for example, according to the procedure described below.

Human kidneys are extracted with an ammonium thiocyanate solution in the usual manner. This extract is acidified to form a precipitate, which is extracted again with a buffer solution. The resulting extract is purified by passing it through one or more columns selected from the group consisting of a column of an ion exchanger, a column of L-arginine or an arginine derivative supported on a carrier, a column of a hemagglutinin supported on a carrier, and a column of a carrier having molecular sieve properties.

Although other features of the thrombolytic composition of the present invention will become apparent from the examples given later, it should be noted that the plasminogen activator constituting the active ingredient thereof is derived from normal cells. It is known that a plasminogen activator derived from malignant tumor cells also has thrombolytic activity. From the nature of a drug administered to human beings, however, all possible risks associated with its administration over a long period of time and/or in large doses must be taken into consideration. In this respect, it may be said that the safety of the thrombolytic composition of the present invention is secured in its origin. Moreover, unlike urokinase, the plasminogen activator constituting the active ingredient of the thrombolytic composition of the present invention has a specific affinity for fibrin, as observed in the natural plasminogen activator derived from blood, so that it can produce very satisfactory results in the treatment of thrombosis. In addition, the thrombolytic composition of the present invention does ot exhibit side effects such as internal hemorrhage, anaphylactic shock and the like. In the basis of these facts, the thrombolytic composition of the present invention is considered to be a very effectual remedy for thrombosis.

The thrombolytic composition of the present invention may be administered to patients in any suitable doses, and can exhibit latent and effective thrombolytic activities accompanied with slight or no side effects. More specifically, the thrombolytic compositions of the present invention can be used in substantially the same manner as common drugs of this type. For example, the plasminogen activator of the present invention may be dissolved in physiological saline containing 5% of human serum albumin and injected. The dose for each injection is preferably 10,000 IU (approximately 100 μg) as expressed in terms of urokinase.

The thrombolytic composition of the present invention is useful in the treatment of acute or chronic occlusion of various vascular beds by a thrombus, as encountered in cases of deep venous thrombosis, pulmonary embolism, myocardial infraction, arterial occlusion, extracorporeal circulation and arteriovenous occlusion.

The present invention is further illustrated by the following examples.

Example 1

[Isolation and purification of the plasminogen activator constituting the active ingredient of the thrombolytic composition of the present invention]

Human kidney tissue was degreased with acetone and then reduced to powder. To 100 g of this powder was added 500 ml of a 2M ammonium thiocyanate solution buffered at pH 7.4 with 0.02M Tris-HCl. The resulting mixture was stirred at 4°C for 10 hours and then centrifuged at 13,000xg for 30 minutes to separate it into a supernatant and a precipitate.

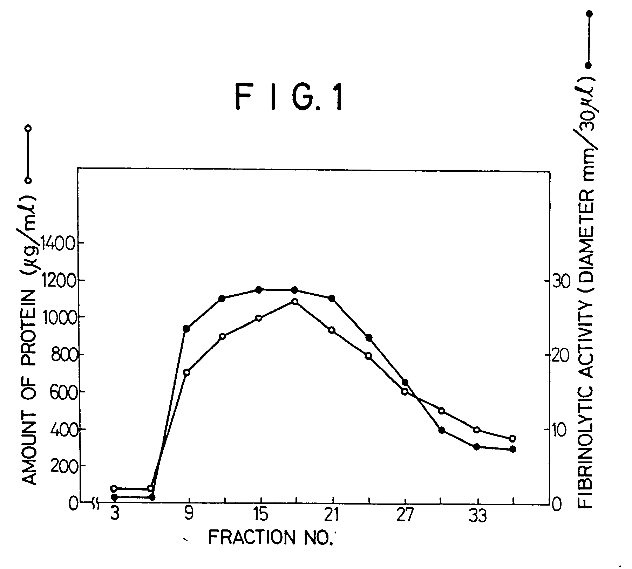

The supernatant was adjusted to pH 3.0 with 1N HCl and the precipitate so formed was collected by centrifugation at 13,000xg for 30 minutes. This precipitate was dissolved in 500 ml of a 0.01M sodium phosphate buffer solution (pH 7.4) containing 0.3M NaCl, 5 mM ε-aminocaproic acid and 0.01% Tween 80 (nonionic surface active agent), which buffer solution will hereinafter be referred to as buffer solution A. To the resulting solution was added 100 ml of fibrin-Celite. This mixture was stirred at 4°C for 30 minutes and then centrifuged at 3,000 mg for 10 minutes. The precipitate was resuspended in 100 ml of buffer solution A. This suspension was packed into a 4 cm × 10 cm column and then washed with 500 ml of buffer solution A. Thereafter, the desired plasminogen activator was eluted with buffer solution A containing 0.4M arginine. The eluate was collected in 2.5 ml fractions. The results thus obtained are shown in Fig. 1 and Table 1. This purification procedure gives a recovery of approximately 30% or higher and makes use of the strong affinity for fibrin of the plasminogen activator constituting the active ingredient of the thrombolytic composition of the present invention.

## Table 1

| Step | Amount of protein (mg) | Total activity (%) |
|------|------------------------|--------------------|
| Extraction with an ammonium thiocyanate solution | 4,500 | 100 |
| Precipitation with an acid | 2,800 | 65 |
| Purification with fibrin-Celite | 26 | 32 |

### Example 2

[Comparison of the thrombolytic effect of the plasminogen activator of the present invention with that of urokinase]

The thrombolytic effect of the plasminogen activator constituting the active ingredient of the thrombolytic composition of the present invention was compared with that of urokinase according to Chandler's loop method. Experiments were made on human blood obtained from healthy male volunteers. Accurately 5 ml of blood was collected and 0.5 ml of a 3.1% sodium citrate solution was added thereto for the purpose of preventing blood coagulation. Moreover, 50 μl of [125]I-labelled fibrinogen was added thereto. After intimate mixing, 1.5 ml portions of the mixture were taken and placed in Tygon tubes having a length of 28 cm and an internal diameter of 0.36 cm. After the addition of 20 mM calcium chloride, both ends of each tube were joined together to form a loop. This looped circulating tube was fixed on a turntable revolving at a speed of 17 rpm having an incline of 23 degrees. After the blood was allowed to clot at room temperature (24—25°C) for 1 hour or 24 hours, the tube was opened and a 10 μl sample was removed and analyzed for radioactivity. Subsequently, a plasminogen activator was added to the tube and both ends thereof were joined together. Then, the looped tube was revolved again on the turntable. After a predetermined time, a 10 μl sample was taken and analyzed for radioactivity. The activity of the plasminogen activator was expressed as the amount of [125]I released from the clot.

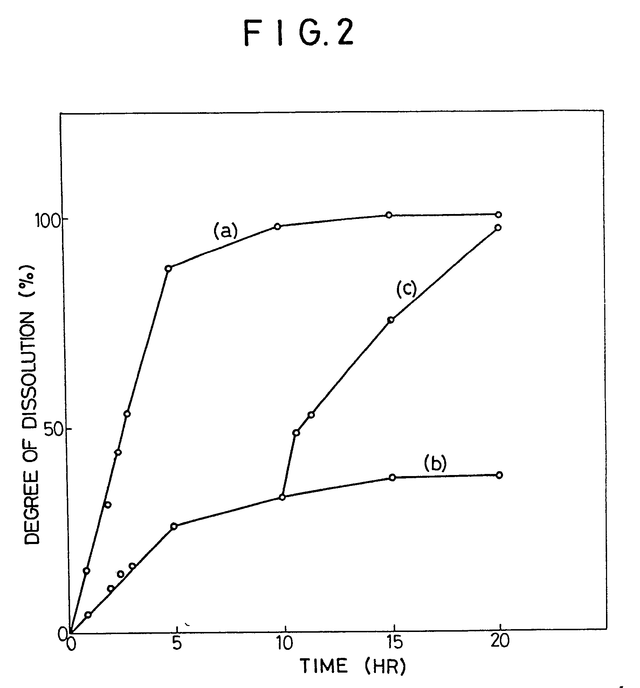

The [125]I-fibrinogen added to the tube was incorporated into the polymer of the clot with an efficiency of approximately 95—98%. However, the clots obtained after 1 hours and 24 hours differed in the degree of cross linking. That is, a partially cross linked (PXL) clot was obtained after 1 hour and a totally cross linked (TXL) one after 24 hours. Fig. 2 illustrates the degree of dissolution when the plasminogen activator constituting the active ingredient of the thrombolytic composition of the present invention or urokinase was allowed to act on a PXL clot. In Fig. 2, curves (a) and (b) show the results obtained with the plasminogen activator of the present invention and urokinase, respectively, indicating that the plasminogen activator of the present invention dissolved the clot much more efficiently than urokinase. Moreover, after 10 hours of reaction, the plasminogen activator of the present invention was added to the system of curve (b) in the same dose as used in the system of curve (a). This induced almost complete dissolution of the clot, as shown by curve (c). In Fig. 2, each dot represents the average of triplicate measurements. More specifically, when used as in a concentration of $1 \times 10^{-9}$ M, the plasminogen activator of the present invention almost completely dissolved the PXL clot in 20 hours, but urokinase dissolved only about 40% of the PXL clot. When the plasmonogen activator of the present invention was added to the latter system after 10 hours of reaction, almost complete release of [125]I was observed. This indicates that the plasminogen activator of the present invention has powerful thrombolytic acitivity.

In experiments using an TXL clot, urokinase dissolved only 35% of the clot in 20 hours, but the plasminogen activator constituting the active ingredient of the thrombolytic composition of the present invention dissolved 90% of the clot. On the other hand, a control experiment was carried out without adding any plasminogen activator. Then, no release of [125]I was detected, indicating that the dissolution of the clot depended on the plasminogen activator.

Example 3

[Effects of the plasminogen activator of the present invention on blood clots of various animals]

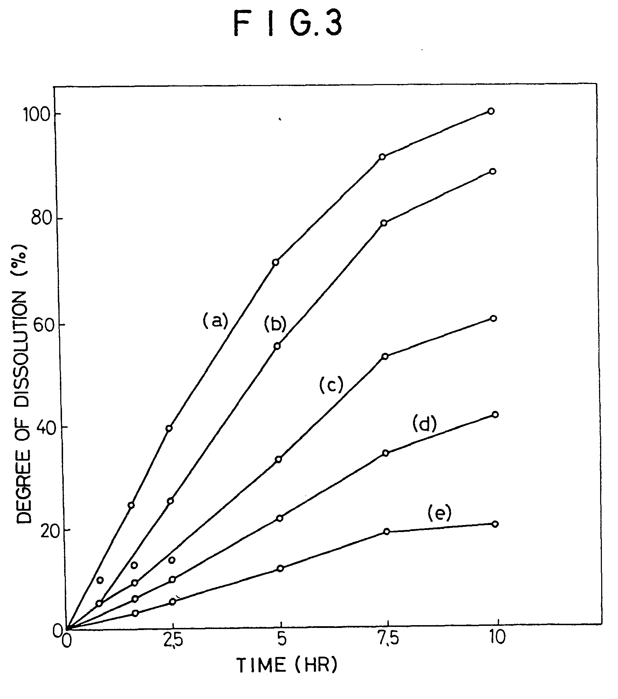

In substantially the same manner as described in Example 2, blood was collected from animals of various species and allowed to clot for 1 hour. The thrombolytic activity of the plasminogen activator constituting the active ingredient of the thrombolytic composition of the present invention was tested by allowing it to act on the partially cross linked (PXL) clots in the plasma of the respective animals. In each experiment, the plasminogen activator was used in a concentration of 20 IU/ml. Until 10 hours after the commencement of the reaction, the degree of dissolution was evaluated by mea) uring the release of [125]I from the clot. Also in this system, the level of radioactivity detected without addition of the plasminogen activator was similar to the background level. That is, only 2—3% of the added radioactivity was detected even after 10 hours. In contrast, approximately 96% of the clot was dissolved in the system using human blood. Fig. 3 illustrates the results obtained by evaluating the degree of dissolution in the systems using blood obtained from man and various animals. In Fig. 3, curves (a), (b), (c), (d) and (e) show the results obtained with human, monkey, rabbit, dog and rat blood. As can be seen from Fig. 3, the degree of dissolution varied according to the animal species. For example, the plasminogen activator of the present invention acted on the clot of monkey blood to substantially the same extent as on the clot of human blood, but exerted only a slight dissolving effect on the clot of rat blood. This suggests that the plasminogen activator constituting the active ingredient of the thrombolytic composition of the present invention is a specific enzyme for human beings and monkeys.

## Claims

1. A thrombolytic composition comprising a plasminogen activator as the active ingredient derived from human kidney and a pharmaceutically acceptable carrier, the plasminogen activator having the following characteristic properties:

(1) The principal protein band obtained by sodium dodecyl sulfate-polyacrylamide gel electrophoresis has an apparent molecular weight of 55,000±1,000;

(2) The apparent molecular weight remains unchanged in the presence of a reducing agent;

(3) the principal band obtained by isoelectric-point electrophoresis has a pI in the range of 7 to 9;

(4) The plasminogen activator has the immunological property of not being adsorbed by anti-urokinase IgG-Sepharose affinity chromatography; and

(5) The plasminogen activator hydrolyzes H-D-valyl-L-leucyl-L-lysine-p-nitroanilide dihydrochloride and H-D-isoleucyl-L-prolyl-L-arginine-p-nitroanilide dihydrochloride, but does not hydrolyse Boc-L-valyl-L-prolyl-L-arginine-4-methylcoumaryl-7-amide, carbobenzoxy-L-phenylalanyl-L-arginine-4-methylcoumaryl-7-amide, L-propyl-L-phenylalanyl-L-arginine-4-methylcoumaryl-7-amide or glutarylglycyl-L-arginine-4-methylcoumaryl-7-amide.

2. A thrombolytic composition as claimed in claim 1 which is useful in the treatment of acute or chronic occlusion of an artery or vein by a thrombus.

## Patentansprüche

1. Thrombolytische Zusammensetzung, die einen Plasminogen-Aktivator als aktiven Bestandteil, der von menschlicher Niere abgeleitet ist, und einen pharmazeutisch verträglichen Träger umfaßt, wobei der Plasminogen-Aktivator die folgenden charakteristischen Eigenschaften aufweist:

(1) Die Hauptproteinbande, die durch Natriumdiodecylsulfat-polyacrylamidgel-Elektrophorese erhalten worden ist, besitzt ein scheinbares Molekulargewicht (relative Molekülmasse) von 55000 ± 1000;

(2) Das scheinbare Molekulargewicht bleibt unverändert in Anwesenheit eines Reduzierungsmittels;

(3) Die Hauptbande, die durch Isoelektrischer-Punkt-Elektrophorese erhalten worden ist, besitzt einen pI in dem Bereich von 7 bis 9;

(4) Der Plasminogen-Aktivator besitzt die immunologische Eigenschaft, daß er durch Anti-Urokinase-IgG-Sepharose-Affinitätschromatographie nicht adsorbiert wird, und

(5) Der Plasminogen-Aktivator hydrolysiert H-D-Valyl-L-leucyl-L-lysin-p-nitroaniliddihydrochlorid und H-D-Isoleucyl-L-prolyl-L-arginin-p-nitroaniliddihydrochlorid, aber er hydrolysiert nicht Boc-L-valyl-L-prolyl-L-arginin-4-methylcumaryl-7-amid, Carbobenzoxy-L-phenylalanyl-L-arginin-4-methylcumaryl-7-amid, L-Prolyl-L-phenylalanyl-L-arginin-4-methylcumaryl-7-amid oder Glutarylglycyl-L-arginin-4-methylcumaryl-7-amid.

2. Thrombolytische Zusammensetzung nach Anspruch 1, die bei der Behandlung von akutem oder chronischem Verschluß einer Arterie oder Vene durch einen Thrombus einsetzbar ist.

## Revendications

1. Composition thrombolytique comprenant un activateur du plasminogène comme ingrédient actif dérivé de rein humain et un véhicule acceptable du point de vue pharmaceutique, l'activateur du plasminogène ayant les propriétés caractéristiques suivantes:

(1) la bande protéique principale obtenue par électrophorèse sur gel de polyacrylamide-dodécylsulfate de sodium a un poids moléculaire apparent de 55000 ± 1000;

(2) le poids moléculaire apparent demure inchangé en présence d'un agent réducteur;

(3) la bande principale obtenue par électrophorèse au point isoélectrique a un pl dans la gamme de 7 à 9;

(4) l'activateur du plasminogène a la propriété immunologique de ne pas être adsorbé par chromatographie d'affinité sur Sepharose-IgG anti-urokinase; et

(5) l'activateur du plasminogène hydrolyse le dichlorhydrate du H-D-valyl-L-leucyl-L-lysine-p-nitroanilide et le dichlorhydrate du H-D-isoleucyl-L-prolyl-L-arginine-p-nitroanilide mais n'hydrolyse pas le Boc-L-valyl-L-propyl-L-arginine-4-méthylcoumaryl-7-amide, le carbobenzoxy-L-phénylalanyl-L-arginine-4-méthylcoumaryl-7-amide, le L-propyl-L-phénylalanyl-L-arginine-4-méthylcoumaryl-7-amide ou le glutaryl-glycyl-L-arginine-4-méthylcoumaryl-7-amide.

2. Composition thrombolytique selon la revendication 1 qui est utile dans le traitement de l'occlusion aiguë ou chronique d'une artère ou d'une veine par un thrombus.

# F I G. 1

FIBRINOLYTIC ACTIVITY ( DIAMETER mm/30 $\mu\ell$ ) ●——●

AMOUNT OF PROTEIN ( $kg/m\ell$ ) o——o

FRACTION NO.

# F I G. 2

# F I G.3